(19) Europäisches Patentamt
European Patent Office
Office européen des brevets

(11) Veröffentlichungsnummer: **0 607 527 A1**

# (12) EUROPÄISCHE PATENTANMELDUNG

(21) Anmeldenummer: **93118701.7**

(22) Anmeldetag: **20.11.93**

(51) Int. Cl.5: **C07C 309/17**, A61K 7/075

(30) Priorität: **19.01.93 DE 4301197**

(43) Veröffentlichungstag der Anmeldung: **27.07.94 Patentblatt 94/30**

(84) Benannte Vertragsstaaten: **BE DE ES FR GB IT NL**

(71) Anmelder: **REWO Chemische Werke GmbH**
**Industriegebiet West**
**D-36396 Steinau(DE)**

(72) Erfinder: **Euler, Axel**
**Brüder-Grimm-Strasse 104**
**D-36396 Steinau an der Strasse(DE)**
Erfinder: **Hamann, Ingo, Dr.**
**Salmünsterer Strasse 13**
**D-63619 Bad Orb(DE)**
Erfinder: **Irrgang, Bernhard, Dr.**
**Erfurter Strasse 2**
**D-63628 Bad Soden-Salmünster(DE)**
Erfinder: **Köhle, Hans-Jürgen, Dr.**
**Lotichiusstrasse 13**
**D-36381 Schlüchtern(DE)**
Erfinder: **Möller, Christl**
**Bergwinkelstrasse 2**
**D-36396 Steinau an der Strasse(DE)**
Erfinder: **Schiesser, Hans-Georg**
**Reichenaustrasse 6**
**D-63628 Bad Soden-Salmünster(DE)**

## (54) Poly(oxyalkylen)alkanolaminfettsäureester, Verfahren zu ihrer Herstellung und ihre Verwendung.

(57) Die Erfindung betrifft Poly(oxyalkylen)alkanolaminfettsäureester der allgemeinen Formel (1)

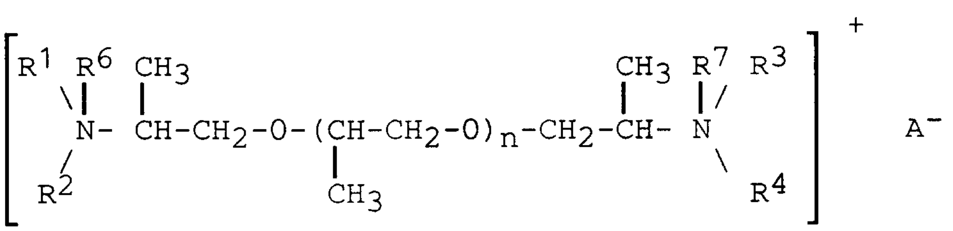

worin
$R^1$, $R^2$, $R^3$, $R^4$ gleich oder verschieden die Reste $R^5$-(O-$CH_2$-$CH_2$)$_m$- bedeuten mit
$R^5$ = gegebenenfalls substituierter, gegebenenfalls Mehrfachverbindungen enthaltender Acylrest mit 6 -22 mit
$R^5$ = Sulfosuccinatrest der Formeln

-C(O)-CH($SO_3^-X^+$)-CH2-$COO^-X^+$

oder

-C(O)-$CH_2$-CH-($SO_3^-X^+$)$COO^-X^+$

oder mit $R^5$ = H

EP 0 607 527 A1

und worin $R^5$ mindestens einmal ein Acylrest und mindestens einmal ein Sulfosuccinatrest ist, $R^6$, $R^7$ gleich oder verschieden H, $-CH_3$, $-C_2H_5$
sein können

n = 2 - 8,
m = 1 - 5 beträgt
$A^-$ = mindestens ein organisches oder anorganisches Anion ist, wenn R6 und/oder R7 ungleich H ist,
$X^+$ = H, Alkalimetall-, Ammonium-Ion ist.

Gegenstand der Erfindung sind neue Sulfosuccinate auf Basis von Poly(oxyalkylen)-alkanolaminfettsäureestern und ein Verfahren zu ihrer Herstellung und ihre Verwendung in wäßrigen Zubereitungen.

Kosmetische Präparate, insbesondere solche, die in den Bereich der Haar- und Körperreinigung fallen, wie Duschbäder, Schaumbäder, Haarshampoos, flüssige Seifen, enthalten als Reinigungskomponenten hauptsächlich Aniontenside wie Carboxylate, Alkylsulfate und Alkylethersulfate, Sulfosuccinate.

Diese Zubereitungen sollen die Hautoberfläche reinigen, vorzugsweise nur den auf ihr anhaftenden Film, welcher aus Körperausscheidungen wie Schweiß und Fetten, Hautschuppen oder abgelagertem Schmutz aus der Umgebung bestehen kann. Die Reinigungsmittel sollen keinesfalls die Haut auslaugen, sie reizen oder ihre natürliche Funktion beeinträchtigen.

Da die Präparate aber bei ihrer häufigen - in den letzten Jahren fast täglichen - Anwendung zu Irritationen der Haut führen können, werden zur Verbesserung der Haut- und Augenschleimhautverträglichkeit häufig sogenannte milde Tenside mitverwendet, wie beispielsweise Betaine, Proteinderivate, Ampholyte, Alkylethercarboxylate und Sulfosuccinate.

Insbesondere für Babypflegemittel und Babyshampoos wird besonderer Wert auf extrem niedrige Gehalte an die Haut und Augenschleimhaut reizende Substanzen gelegt. Die konventionell wegen ihrer ausgezeichneten Reinigungs- und Schaumbildungseigenschaften verwendeten anionischen Tenside können durch die bekannten milden Co-Tenside in ihrer Irritationswirkung zwar weitgehend gemildert werden, in der Praxis werden aber noch Verbesserungen insbesondere in Richtung auf Augenschleimhautverträglichkeit gefordert.

In hohen Konzentrationen oder allein sind die milden Tenside zwar weitgehend irritationsfrei, zeigen dann jedoch keine praxisgerechten Schäumungs- und Reinigungseigenschaften und unbefriedigende Viskositäten.

Ein weiteres Kriterium für die Brauchbarkeit der oberflächenaktiven Substanzen ist ihre Toxizität. Die Toxizität liegt in den Tensiden selbst oder ihren durch Wechselwirkung mit den Bestandteilen der Rezeptur entstehenden Produkten.

So werden die wegen ihrer vorteilhaften anwendungstechnischen und rückfettenden Eigenschaften bislang gern mitverwendeten Fettsäurealkanolamide in letzter Zeit nur noch selten in kosmetischen Präparaten eingesetzt, da der bei der Herstellung verbleibende Restgehalt an freiem Diethanolamin nicht ausgeschlossen werden und damit Anlaß zur Nitrosaminbildung sein kann (EP-A-0 306 843).

Aufgrund des gewachsenen Risiko- und Umweltbewußtseins werden daher Produkte verlangt, welche eine nicht oder kaum nachweisbare Toxizität aufweisen.

Die in der Praxis eingeführten Sulfosuccinate auf Basis von Alkoholen bzw. ethoxilierten Alkoholen weisen zwar eine gute Reinigungswirkung und Schaumkraft auf, sind jedoch in der Hautverträglichkeit verbesserungswürdig.

Aufgabe der vorliegenden Erfindung ist es, ultramilde hautfreundliche Reinigungsmittel für Haushalt und Industrie und kosmetische Präparate, insbesondere solche, die in den Bereich der Haar- und Körperreinigung und der Babypflege fallen, zur Verfügung zu stellen.

Diese Aufgabe wird gelöst durch die erfindungsgemäß hergestellten Poly(oxyalkylen)-alkanolaminfettsäureester sowie deren wäßrige Lösungen.

Gegenstand der Erfindung sind Poly(oxyalkylen)alkanol-aminfettsäureester der allgemeinen Formel (1)

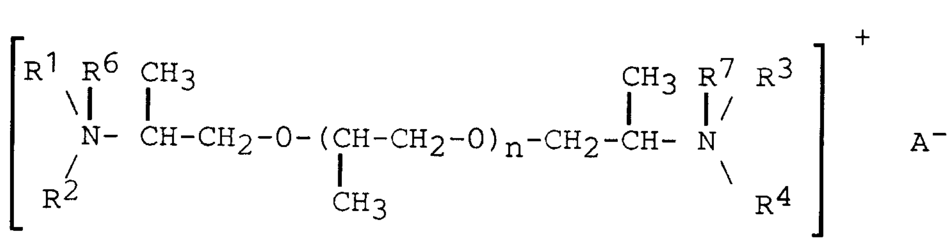

worin

$R^1$, $R^2$, $R^3$, $R^4$ gleich oder verschieden die Reste $R^5$-$(O\text{-}CH_2\text{-}CH_2)_m$- bedeuten mit

$R^5$ = gegebenenfalls substituierter, gegebenenfalls Mehrfachverbindungen enthaltender Acylrest mit 6 - 22, vorzugsweise 8 - 18 C-Atomen, mit

$R^5$ = Sulfosuccinatrest der Formeln

-C(O)-CH($SO_3^-X^+$)-CH2-$COO^-X^+$

oder

-C(O)-$CH_2$-CH-($SO_3^-X^+$)$COO^-X^+$

oder mit $R^5$ = H
und worin $R^5$ mindestens einmal ein Acylrest und mindestens einmal ein Sulfosuccinatrest ist, $R^6$, $R^7$ gleich oder verschieden H, -$CH_3$, -$C_2H_5$ sein können

- n = 2 - 8, vorzugsweise 4 - 6
- m = 1 - 5, vorzugsweise 2 - 4 und die Summe aller m vorzugsweise 8 - 15 beträgt,
- $A^-$ = mindestens ein organisches oder anorganisches Anion ist, wenn R6 und/oder R7 ungleich H ist,
- $X^+$ = H, Alkalimetall-, Ammonium-Ion ist.

Ein weiterer Gegenstand der Erfindung ist ein Verfahren zur Herstellung der erfindungsgemäßen Poly(oxyalkylen)alkanolaminfettsäureester, welches dadurch gekennzeichnet ist, daß Aminverbindungen der allgemeinen Formel (2)

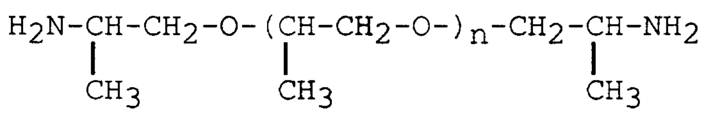
(2)

worin n = 2 - 8, vorzugsweise 4 - 6 ist, mit 4 - 20, vorzugsweise 8 - 15 mol, Ethylenoxid bei 140 - 160 °C, gegebenenfalls unter Mitverwendung eines Katalysators, bei 1 - 4 bar in einem Druckreaktor zu Verbindungen der allgemeinen Formel (3)

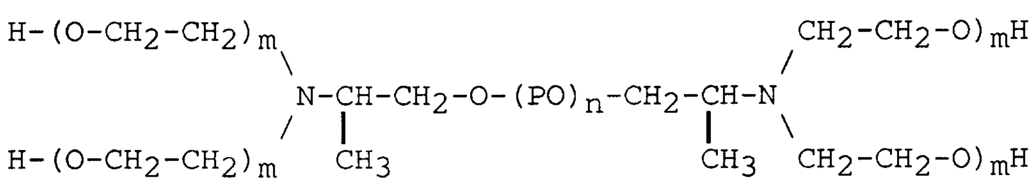

worin PO der Rest -CH($CH_3$)-$CH_2$-O-ist und n und m die oben angegebene Bedeutung haben, umgesetzt werden und mit ≧1 Mol Fettsäure(alkylester) pro Mol der Verbindung Formel (3) bei 160 - 240 °C verestert und das sich bildende Kondensationswasser bzw. der Alkohol kontinuierlich abdestilliert, gegebenenfalls zur Vervollständigung der Reaktion ein Unterdruck angelegt wird und dieser Ester in einer Folgereaktion mit ≧1 Mol Maleinsäureanhydrid bei 70 - 80 °C zu dem entsprechenden Maleinsäurehalbester umgesetzt wird, welches in letzter Stufe mit 1 Equivalent, bezogen auf Maleinsäurehalbester, wäßriger $X_2SO_3$-Lösung, worin X die Bedeutung Alkalimetall oder Ammonium hat, bei 60 - 80 °C zum Sulfobernsteinsäurehalbester reagiert.

Ein weiterer Gegenstand der Erfindung sind wäßrige Zubereitungen, enthaltend

a) 1 - 10 Gew.-Teile, vorzugsweise 2 - 7 Gew.-Teile, mindestens einer der Verbindungen der allgemeinen Formel (1)

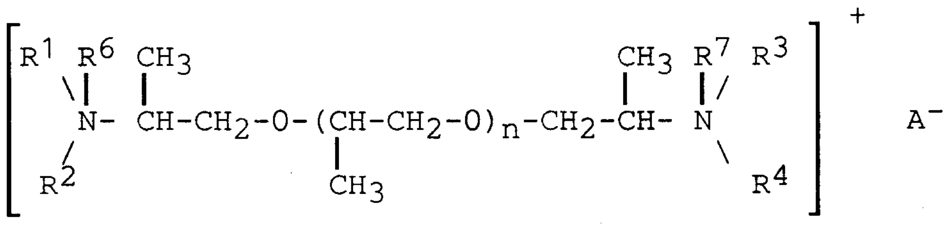

worin

$R^1$, $R^2$, $R^3$, $R^4$ gleich oder verschieden die Reste $R^5$-(O-$CH_2$-$CH_2$)m- bedeuten mit

$R^5$ = gegebenenfalls substituierter, gegebenenfalls Mehrfachverbindungen enthaltender Acylrest mit 6 - 22, vorzugsweise 8 - 18 C-Atomen, mit

$R^5$ = Sulfosuccinatrest der Formeln

-C(O)-CH($SO_3$-X + )-$CH_2$-COO-X +

oder

-C(O)-$CH_2$-CH-($SO_3^-X^+$)-$COO^-X^+$

oder mit $R^5$ = H

und worin $R^5$ mindestens einmal ein Acylrest und mindestens einmal ein Sulfosuccinatrest ist, $R^6$, $R^7$ gleich oder verschieden H, -$CH_3$, -$C_2H_5$ sein können

n = 2 - 8, vorzugsweise 4 - 6

m = 1 - 5, vorzugsweise 2 - 4 und die Summe aller m vorzugsweise 8 - 15 beträgt,

$A^-$ = mindestens ein organisches oder anorganisches Anion ist, wenn $R^6$ und/oder $R^7$ ungleich H ist,

$X^+$ = H, Alkalimetall-, Ammonium-Ion ist,

b) 1 - 20 Gew.-Teile, vorzugsweise 2 - 16 Gew.-Teile, mindestens eines Tensids aus der Gruppe der nichtionischen, amphoteren, zwitterionischen, ionischen Tenside und gegebenenfalls

c) 0,1 - 10 Gew.-Teile Verdickungsmittel, Duftstoffe, Pflanzenextrakte und sonstige Zusatz- und Hilfsstoffe und

d) ad 100 Wasser.

Ein weiterer Gegenstand der Erfindung sind Babyshampoos, enthaltend 1 - 10, insbesondere 2 - 7 Gew.-Teile mindestens einer der Verbindungen der allgemeinen Formel (1).

Ein weiterer Gegenstand der Erfindung sind wäßrige Haarreinigungsmittel, enthaltend 1 - 10, insbesondere 2 - 7 Gew.-Teile mindestens einer der Verbindungen der allgemeinen Formel (1).

Weitere Gegenstände der Erfindung sind durch die Ansprüche gekennzeichnet.

Die erfindungsgemäß mitverwendeten Verbindungen der allgemeinen Formel (1) sind herstellbar aus handelsüblichen Poly(oxyalkylen)polyaminen (Jeffamine der Firma Texaco), welche nach an sich bekannten Verfahren in erster Stufe ethoxyliert, anschließend mit Monocarbonsäuren verestert und weiter mit Maleinsäureanhydrid zu den entsprechenden Halbestern umgesetzt werden. Die Reaktion der Halbester zu Sulfosuccinaten erfolgt in einer letzten Stufe mit Sulfiten nach an sich ebenfalls bekannten Verfahren (DE-OS 27 00 072). Die Reaktionsprodukte liegen im allgemeinen als 30 - 60 Gew.-%ige Lösungen vor und sind ohne weitere Reinigungs- und/oder Konzentrationsstufen direkt weiter verwendbar.

Weitere Gegenstände der Erfindung sind durch die Ansprüche gekennzeichnet.

Die erfindungsgemäß mitverwendeten Poly(oxyalkylen)amine der allgemeinen Formel (2) sind handelsübliche Produkte, welche nach bekannten Verfahren durch die Umsetzung von Polyoxyalkylenalkoholen mit Ammoniak unter Druck hergestellt werden.

Als Fettsäuren werden die einbasischen Säuren mit 8 - 22 C-Atomen - bevorzugt die natürlich vorkommenden - verwendet wie Laurinsäure, Palmitinsäure, Stearinsäure, Ölsäure, Linolsäure, Linolensäure, Ricinolsäure, Kokosfettsäure oder deren Mischungen (vgl. J. Am. Oil Chem. Soc. 38, 517-520 (1961) und EP-A-0 190 779).

Die Reaktion zum Sulfosuccinat erfolgt mit Maleinsäureanhydrid und anschließender Sulfierung mit Natriumsulfit nach an sich bekannten Verfahren (DE-OS 27 00 072).

Die erfindungsgemäßen Verbindungen haben Alkoxylierungsgrade (m) von jeweils 1 - 15, vorzugsweise 2 - 4, wobei die Summe aller Alkoxylierungseinheiten vorzugsweise 8 - 15 beträgt. Diese Werte sind als statistische Mittelwerte zu betrachten.

Für die erfindungsgemäße Verwendung ist eine Reinigung nicht erforderlich, das heißt, die technischen 30 - 60 %igen wäßrigen Lösungen sind als solche direkt weiterverwendbar.

Sie sind wertvolle ultramilde Rezepturbestandteile für Cremes, Emulsion, Gele und können zur Anpassung an den vorgesehenen Anwendungszweck noch die jeweils üblichen Hilfs- und Zusatzstoffe enthalten, welche für die Herstellung von Reinigungsmitteln und kosmetischen Präparaten im Bereich der Haar- und Körperreinigung, das heißt für Duschbäder, Schaumbäder, Shampoos, flüssige Seifen, Babypflege- und -waschmittel sowie für milde Reinigungsmittel wie Geschirrspülmittel, Allzweckreiniger oder Neutralreiniger für manuelle Anwendung üblich sind, mitverwendet werden.

Zur Anpassung an den vorgesehenen Anwendungszweck können noch die jeweils üblichen Hilfs- und Zusatzstoffe, welche für die Herstellung von kosmetischen Präparaten im Bereich der Haar- und Körperreinigung, das heißt für Duschbäder, Schaumbäder, Shampoos, flüssige Seifen, Babypflege- und -waschmittel sowie für milde Reinigungsmittel wie Geschirrspülmittel für manuelle Anwendung, Neutralreiniger und Allzweckreiniger üblich sind, mitverwendet werden.

Ein weiterer Vorteil liegt darin, daß bei der gleichzeitigen Anwesenheit von anionischen und kationischen Verbindungen in einer Rezeptur diese sowohl reinigende als auch pflegende Wirkung entfaltet.

Außerdem kommen diese Rezepturen der modernen Auffassung nach einem mehrseitig anwendbaren Mittel entgegen, da sie außer zur Haarreinigung und gleichzeitiger Haarpflege auch reinigend und pflegend auf die Haut wirken. Bei Mitverwendung der Verbindungen der allgemeinen Formel (1) lassen sich also die Forderungen der Praxis nach Body/Hair-Care-Shampoos problemlos erfüllen.

Für die kosmetischen Anwendungen können die auf diesen Gebieten üblichen Tenside, Duftstoffe, Konservierungsmittel, Farbstoffe, Pflanzenextrakte bzw. sonstige kosmetischen Additive mitverwendet werden.

Neben den erfindungsgemäßen Verbindungen, welche in Mengen von 1 - 10 Gew.-Teilen, insbesondere 2 - 8 Gew.-Teilen bei Shampoos sowie 1 - 5, vorzugsweise 1,5 - 3 Gew.-Teilen bei Reinigungsmitteln eingesetzt werden, werden die anderen Tenside bei den Shampoos üblicherweise in Mengen von 1 - 20 Gew.-Teilen, vorzugsweise 5 - 15 Gew.-Teilen, mitverwendet.

Als Verdickungsmittel werden 1 - 8 Gew.-Teile der auf diesem Gebiet üblichen Verbindungen wie Glycerinfettsäureesterethoxylate, Fettalkoholethoxylate, Fettsäurealkylolamide und die üblichen Alkali-, Erdalkali- und Ammoniumsalze, welche bei 20 °C in Mengen von mindestens 1 Gew.-% in Wasser löslich sind, wie insbesondere NaCl, $NH_4Cl$ eingesetzt.

Die in den nachfolgenden Beispielen angewandten Analysenmethoden sind die auf diesem Gebiet üblichen und sind im einzelnen:

Feststoffgehalt:

Der Gehalt wird bestimmt durch Erhitzen und Trocknen der Substanz bis zur Gewichtskonstanz bei 105 °C.

Verseifungszahl (VZ):

Die Verseifungszahl ist ein Maß für die in Fetten und technischen Fettsäuren enthaltenen freien und gebundenen Säuren. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): DGF C-V3.

Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).
Die Tertiäraminzahl gibt die Anzahl Milligramm Kaliumhydroxid an, die der Tertiäraminbasizität von 1 Gramm der Aminverbindung äquivalent sind (mg KOH/g).
Die Werte werden bestimmt nach American Oil Chemists Society (A.O.C.S.) Official Method Tf 2a-64.

Gehalt an Kationaktiv-Substanz (cat. $SO_3$):

Diese Methode dient zur Bestimmung des Gehalts an kationenaktiven Substanzen. Kationenaktive Substanzen sind hier langkettige Verbindungen, welche quaternäre Ammoniumgruppen enthalten. Der Gehalt wird angegeben in Prozent quaternäre Verbindung, berechnet als SO3-Äquivalent mit einem Molekulargewicht von 80 g/mol.
Der Gehalt wird bestimmt durch eine Zweiphasentitration gemäß ISO-Norm 2871-1 und 2871-2 (1988 E).

Analysenmethoden

**Härteverträglichkeit:** DIN 53 905
**Hautverträglichkeit: Zein-Test:**
Götte, Ernst, Chem. Phys. Appl. Surface Act. Subst. Proc. Int. Congr. **4** (1964) 83-90.
< 200 mg N/ 100 ml = nicht irritierend
200 - 400 mg N / 100 ml = leicht irritierend
> 400 mg N/ 100 ml = irritierend
**Hautverträglichkeit: RBC-Test:**
Pape, Hoppe, Drug. Res. **40 (I), Nr.4** (1990), 498.
L/D-Wert: > 100 = non irritant
> 10 = slightly irritant
> 1 = moderately irritant
> 0.3 = irritant
< 0.3 = very irritant
< 0.1 = severely irritant
**Oberflächenspannung:**
Dr. R. Hensch, Fette, Seifen, Anstrichmittel, **72** (1970), S. 969 - 977.
**Viskosität:**
Brookfield Rotationsviskosimeter (Becher und Spindel), gemessen bei 20 °C nach den Angaben des Geräteherstellers.
Bestimmung des **Schaumvermögens** nach Ross-Miles in Anlehnung an DIN 53 902 Teil 2 mit der Abänderung, daß folgende Maße und Mengen verwendet wurden:

| | |
|---|---|
| Innendurchmesser der Auslaufdüse: | 3.5 mm |
| Fallhöhe der Probelösung: | 940 mm |
| Menge der vorgelegten Probenlösung: | 50 ml |
| Menge der zulaufenden Probenlösung: | 200 ml |
| Meßtemperatur: | 40 °C |
| Lösungsmittel: | demineralisiertes Wasser |

Herstellung der Poly(oxyalkylen)alkanolaminfettsäureester

Beispiel 1:

In einem Laborautoklaven werden 912 g (2 mol) Polyoxyalkylenpolyamin D 400 vorgelegt und auf 150 °C erhitzt. Bei dieser Temperatur werden anschließend 704 g (16 mol) Ethylenoxid eingeleitet, so daß der Druck im Reaktionsgefäß nicht über 4 bar ansteigt. Nach Beendigung der Umsetzung weist das Produkt eine GAZ von 141 und eine TAZ von 141 auf.

In einem 1 Liter-4-Halskolben mit Rührer, Innenthermometer, Inertgaszuleitung und Destillationsbrücke werden 606 g (0,75 mol) POA-Ethoxylat mit 179 g (0,82 mol) Kokosfettsäure $C_{12-18}$ versetzt und unter einer Stickstoffatmosphäre auf 180 °C erhitzt. Das entstehende Reaktionswasser wird kontinuierlich abdestilliert; gegebenenfalls wird die Reaktion durch Anlegen von Vakuum vervollständigt. Das Veresterungsprodukt besitzt eine GAZ von 110 und eine TAZ von 110. Die Verseifungszahl liegt bei 60.

342 g (0,33 mol) POA-Ester werden in einem 500 ml 4-Halskolben mit Rührer, Innenthermometer, Inertgaszuleitung und Rückflußkühler mit 33 g (0,33 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 42 g (0,33 mol) Natriumsulfit in 570 g Wasser gegossen und bei dieser

Temperatur gerührt, bis der $SO_2$-Gehalt der Lösung <0,01 % beträgt. Es entsteht eine Lösung mit einem Feststoffgehalt von 40,2 % und einem kationischen Aktivgehalt von 2,3 %.

Beispiel 2:

302 g (0,25 mol) POA-Ester aus Beispiel 1 werden in einem 500 ml 4-Halskolben mit Rührer, Innenthermometer, Inertgaszuleitung und Rückflußkühler mit 73,5 g (0,75 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 94,5 g (0,75 mol) Natriumsulfit in 820 g Wasser gegossen und bei dieser Temperatur gerührt, bis der $SO_2$-Gehalt der Lösung <0,01 % beträgt. Es entsteht eine Lösung mit einem Feststoffgehalt von 40,7 % und einem kationischen Aktivgehalt von 0,4 %.

Beispiel 3:

In einem 1 Liter-4-Halskolben mit Rührer, Innenthermometer, Inertgaszuleitung und Destillationsbrücke werden 606 g (0,75 mol) POA-Ethoxylat aus Beispiel 1 mit 329 g (1,5 mol) Kokosfettsäure $C_{12-18}$ versetzt und unter einer Stickstoffatmosphäre auf 180 °C erhitzt. Das entstehende Reaktionswasser wird kontinuierlich abdestilliert; gegebenenfalls wird die Reaktion durch Anlegen von Vakuum vervollständigt. Das Veresterungsprodukt besitzt eine GAZ von 93 und eine TAZ von 94. Die Verseifungszahl liegt bei 93.

302 g (0,25 mol) POA-Ester werden in einem 500 ml 4-Halskolben mit Rührer, Innenthermometer, Inertgaszuleitung und Rückflußkühler mit 49 g (0,5 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 63 g (0,5 mol) Natriumsulfit in 625 g Wasser gegossen und bei dieser Temperatur gerührt, bis der $SO_2$-Gehalt der Lösung <0,01 % beträgt. Es entsteht eine Lösung mit einem Feststoffgehalt von 41,6 % und einem kationischen Aktivgehalt von 1,4 %.

Beispiel 4:

302 g (0,25 mol) POA-Ester aus Beispiel 3 werden in einem 500 ml 4-Halskolben mit Rührer, Innenthermometer, Inertgaszuleitung und Rückflußkühler mit 25 g (0,25 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 32 g (0,25 mol) Natriumsulfit in 500 g Wasser gegossen und bei dieser Temperatur gerührt, bis der $SO_2$-Gehalt der Lösung <0,01 % beträgt. Es entsteht eine Lösung mit einem Feststoffgehalt von 39,9 % und einem kationischen Aktivgehalt von 2,4 %.

Beispiel 5

In einem Laborautoklaven werden 456 g (1 mol) Polyoxyalkylenpolyamin D 400 vorgelegt und auf 150 °C erhitzt. Bei dieser Temperatur werden anschließend 528 g (12 mol) Ethylenoxid eingeleitet, so daß der Druck im Reaktionsgefäß nicht über 4 bar ansteigt. Nach Beendigung der Umsetzung weist das Produkt eine GAZ von 113 und eine TAZ von 113 auf.

In einem 1 Liter-4-Halskolben mit Rührer, Innenthermometer, Inertgaszuleitung und Destillationsbrücke werden 738 g (0,75 mol) POA-Ethoxylat mit 329 g (1,5 mol) Kokosfettsäure $C_{12-18}$ versetzt und unter einer Stickstoffatmosphäre auf 180 °C erhitzt. Das entstehende Reaktionswasser wird kontinuierlich abdestilliert; gegebenenfalls wird die Reaktion durch Anlegen von Vakuum vervollständigt. Das Veresterungsprodukt besitzt eine GAZ von 81 und eine TAZ von 81. Die Verseifungszahl liegt bei 80.

347 g (0,25 mol) POA-Ester werden in einem 500 ml 4-Halskolben mit Rührer, Innenthermometer, Inertgaszuleitung und Rückflußkühler mit 49 g (0,5 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 63 g (0,5 mol) Natriumsulfit in 640 g Wasser gegossen und bei dieser Temperatur gerührt, bis der $SO_2$-Gehalt der Lösung <0,01 % beträgt. Es entsteht eine Lösung mit einem Feststoffgehalt von 42,7 % und einem kationischen Aktivgehalt von 1,1 %.

Beispiel 6:

In einem Laborautoklaven werden 456 g (1 mol) Polyoxyalkylenpolyamin D 400 vorgelegt und auf 150 °C erhitzt. Nach Zusatz von 1 g Kaliumhydroxid werden bei dieser Temperatur anschließend 880 g (20 mol) Ethylenoxid eingeleitet, so daß der Druck im Reaktionsgefäß nicht über 4 bar ansteigt. Nach

Beendigung der Umsetzung weist das Produkt eine GAZ von 83 und eine TAZ von 83 auf.

In einem 1 Liter-4-Halskolben mit Rührer, Innenthermometer, Inertgaszuleitung und Destillationsbrücke werden 668 g (0,5 mol) POA-Ethoxylat mit 109 g (0,5 mol) Kokosfettsäure $C_{12-18}$ versetzt und unter einer Stickstoffatmosphäre auf 180 °C erhitzt. Das entstehende Reaktionswasser wird kontinuierlich abdestilliert; gegebenenfalls wird die Reaktion durch Anlegen von Vakuum vervollständigt. Das Veresterungsprodukt besitzt eine GAZ von 71 und eine TAZ von 70. Die Verseifungszahl liegt bei 37.

507 g (0,33 mol) POA-Ester werden in einem 1 l 4-Halskolben mit Rührer, Innenthermometer, Inertgaszuleitung und Rückflußkühler mit 32 g (0,33 mol) Maleinsäureanhydrid versetzt und unter einer Stickstoffatmosphäre 2 h auf 70 - 80 °C erwärmt. Der gebildete Maleinsäurehalbester wird anschließend in eine auf 70 °C erhitzte Lösung von 42 g (0,33 mol) Natriumsulfit in 250 g Wasser gegossen und bei dieser Temperatur gerührt, bis der $SO_2$-Gehalt der Lösung <0,01 % beträgt. Es entsteht eine Lösung mit einem Feststoffgehalt von 67,5 % und einem kationischen Aktivgehalt von 2,2 %.

Tabelle 1

| Beispiel | $R^1$ | $R^2$ | $R^3$ | $R^4$ | $R^5$ | $R^6/R^7$ | $R^8$ = $C_n$ mit $C_n$ | Σm | n |
|---|---|---|---|---|---|---|---|---|---|
| 1 | $EO^1{}_mR5$ | $EO^1{}_mH$ | Sulfos.* | $EO^1{}_mH$ | $C(O)R^8$ | H | 12-18 | 8 | 4 |
| 2 | $EO^1{}_mR5$ | Sulfos.* | Sulfos.* | Sulfos.* | $C(O)R^8$ | H | 12-18 | 8 | 4 |
| 3 | $EO^1{}_mR5$ | $EO^1{}_mH$ | Sulfos.* | $EO^1{}_mR5$ | $C(O)R^8$ | H | 12-18 | 8 | 4 |
| 4 | $EO^1{}_mR5$ | Sulfos.* | $EO^1{}_mR5$ | Sulfos.* | $C(O)R^8$ | H | 12-18 | 8 | 4 |
| 5 | $EO^1{}_mR5$ | Sulfos.* | $EO^1{}_mR5$ | Sulfos.* | $C(O)R^8$ | H | 12-18 | 12 | 4 |
| 6 | $EO^1{}_mR_5$ | $EO^1{}_mH$ | Sulfos.* | $EO^1{}_mH$ | $C(O)R^8$ | H | 12-18 | 20 | 4 |

Sulfos.* = Sulfosuccinatrest der Formel: $EO_m-C(O)-CH(SO_3^-X^+)CH_2-COO^-X^+$ oder $EO_m-C(O)-CH_2-CH(SO_3^-X^+)-COO^-X^+$.

$^1 = -(OCH_2-CH_2)$

Tabelle 2

**Erklärung der in den Rezepturen eingesetzten Tenside nach:**

| CTFA-Namen | Markennamen[1] | Konz. |
|---|---|---|
| 1) Disodium PEG-4 Cocamido MIPA-Sulfosuccinate | REWOPOL SBZ® | 50 % |
| 2) Disodium Lauroamphodiacetate (and) Sodium Lauryl Sulfate (and) Hexylene Glycol | REWOTERIC AM G 30® | 38 % |
| 3) Cocamidopropyl Hydroxysultaine | REWOTERIC AM CAS® | 50 % |
| 4) Ricinoleamidopropyltrimonium Methosulfate | REWOQUAT RTM 50® | 40 % |
| 5) PEG-200 Hydrogenated Glyceryl Palmitate (and) PEG-7 Glyceril Cocoate | REWODERM LI S 80® | 70 % |
| 6) Disodium Laureth Sulfosuccinate | REWOPOL SB FA 30® | 40 % |
| 7) Sodium Laureth Sulfate | REWOPOL NL 3-28® | 28 % |
| 8) Cocamidopropyl Betaine | REWOTERIC AM B 13® | 35 % |
| 9) Disodium Cocoamphodiacetate | REWOTERIC AM 2 C NM® | 50 % |
| 10) PEG-80 Glyceryl Tallowate | REWODERM LI 48-50® | 50 % |
| 11) Cocamidopropyl Betaine | REWOTERIC AM B 14® | 35 % |
| 12) Glyceryl Stearate | REWOPOL PGK 2000® | 35 % |
| 13) Laureth-6 | REWOPAL LA 6® | 100 % |

1 = der Firma REWO Chemische Werke GmbH

Kosmetische Formulierungen

Die Komponenten werden in der in den Beispielen der Grund- bzw. Testrezepturen angegebenen Reihenfolge unter Rühren bei ca. 25 - 30 °C homogenisiert. Dabei wird auf die exakte und vollkommene

Lösung aller Zusatzstoffe geachtet.

Bei Einsatz von Perlglanzmittel (REWOPOL® PGK 2000) ist auf eine exakte Verteilung und Schaumfreiheit zu achten. Die pH-Wert-Einstellung erfolgt mit Citronensäure auf den gewünschten Wert, die Viskositätseinstellung wird mit NaCl durchgeführt.

GRUNDREZEPTUREN

Duschbad

| | |
|---|---|
| Erfindungsgemäße Verbindung | 5 - 20 Gew.-Teile |
| REWOTERIC®* AM G 30[2)] | 15 - 40 " |
| REWOTERIC®* AM CAS[3)] | 4 - 12 " |
| REWOQUAT®* RTM 50[4)] | 2 - 7 " |
| REWODERM®* LI S 80[5)] | 1.5 - 6 " |
| demin. Wasser | ad 100 |

Pflegendes Duschshampoo

| | |
|---|---|
| Erfindungsgemäße Verbindung | 5 - 20 Gew.-Teile |
| REWOPOL®* NL 3-28[7)] | 15 - 45 " |
| REWOTERIC®* AM B 13[8)] | 10 - 35 " |
| demin. Wasser | ad 100 |

Babyshampoo

| | | |
|---|---|---|
| Erfindungsgemäße Verbindung | 5 - 20 | Gew.-Teile |
| REWOTERIC®* AM 2 C NM[9)] | 5 - 20 | " |
| REWOPOL®* NL 3-28[7)] | 5 - 35 | " |
| REWODERM®* LI S 80[5)] | 1.5 - 6 | " |
| REWODERM®* LI 48-50 [10)] | 0 - 5 | |
| demin. Wasser | ad 100 | |

Haarshampoo

| | | |
|---|---|---|
| Erfindungsgemäße Verbindung | 3 - 7 | Gew.-Teile |
| REWOPOL®* NL 3-28[7)] | 30 - 50 | " |
| REWOTERIC®* AM B 13[8)] | 15 - 25 | " |
| REWOPOL®* PGK 2000[13)] | 4 - 6 | " |
| demin. Wasser | ad 100 | |

Hautreinigungsmittel und Make-up-Entferner für empfindliche Haut

| | | |
|---|---|---|
| Erfindungsgemäße Verbindung | 5 - 20 | Gew.-Teile |
| REWOTERIC®* AM B 14[12)] | 5 - 25 | " |
| demin. Wasser | ad 100 | |

Spülmittel

| | | |
|---|---|---|
| Erfindungsgemäße Verbindung | 2 - 13 | Gew.-Teile |
| REWOPOL®* NL 3-28[7)] | 3 - 80 | " |
| REWOTERIC®* AM CAS[3)] | 2 - 10 | " |

| | | |
|---|---|---|
| REWOPOL®* LA 6[14)] | 1 - 10 | " |
| demin. Wasser | ad 100 | |

* = Eingetragenes Wahrenzeichen der Fa. REWO Chemische Werke GmbH, Steinau an der Strasse.

Anwendungstechnische Beispiele

Rezeptur: Pflegendes Duschshampoo

Die erfindungsgemäßen Beispiele der Testrezeptur sind von einem Testpanel von 20 Personen (weiblich und männlich) bewertet worden bezüglich:

- Anschaumvermögen
- Hautgefühl während des Waschens
- Hautgefühl der abgetrockneten Haut

Dabei erfolgt die Bewertung nach einem abgestuften Bewertungssystem, wobei die angeführte Beurteilung den arithmetischen Mittelwert wiedergibt.

Anschaumvermögen: Bestimmung der Menge an gebildetem Schaum beim Verreiben zwischen den feuchten Händen (Händewaschen).

Tabelle 3

**Testrezeptur: Pflegendes Duschshampoo, hautfreundlich**

| **Rezeptur** (Angabe in Gewichtsteilen) | **1** | **2** | **3** | **4** |
|---|---|---|---|---|
| **Beispiel 3** | 5 | - | - | - |
| **Beispiel 4** | - | 5 | - | - |
| **Beispiel 1** | - | - | 5 | - |
| **REWOTERIC® AM B 13**[8)] | 20 | 20 | 20 | 30 |
| **REWOPOL® NL 3-28**[7)] | 40 | 40 | 40 | 44 |
| **demin. Wasser** | 35 | 35 | 35 | 26 |
| **pH-Wert mit Citronensäure eingestellt** | 6.5 | 6.5 | 6.5 | 6.5 |
| **Anschaumvermögen** | weniger gut | **gut** | **gut** | gut |
| **Schaumstruktur** | feinblasig,cremig | grobblasig, cremig | grobblasig, sehr cremig | feinblasig |
| **Hautgefühl nasse Haut** | **gut,cremig** | **gut** | **gut** | etwas stumpf |
| **Hautgefühl trockene Haut** | | | | |
| **- sofort nach Abtrocknen** | glatt, trocken | etw. stumpf, klebrig | etwas stumpf stumpf, | etwas klebrig |
| **- nach kurzer Zeit** | glatt, sehr weich | glatt, sehr weich | glatt, sehr weich | glatt, weich |

Testrezeptur: Haarshampoo

Die erfindungsgemäßem Beispiele der Rezeptur sind von 10 in der Anwendung erfahrenen Testpersonen auf Kämmbarkeit und Volumen des Haares sowie Sitz der Frisur bewertet worden (Tabelle 4)

Tabelle 4

**Haarshampoo, mild, Reduktion der Irritationswerte**

| Rezeptur | 1 | 2 | 3 | 4 |
|---|---|---|---|---|
| Beispiel 1 | 5 | - | - | - |
| Beispiel 5 | - | 5 | - | - |
| Beispiel 3 | - | - | 5 | - |
| REWOTERIC® SBZ 1) | - | - | - | 5 |
| REWOPOL® NL 3-28 7) | 40 | 40 | 40 | 40 |
| REWOTERIC® AM B 13 8) | 20 | 20 | 20 | 20 |
| REWOPAL® PGK 2000 12) | 5 | 5 | 5 | 5 |
| demin. Wasser | 30 | 30 | 30 | 30 |
| Schaumvermögen / mm | 175 / 175 | 190 / 180 | 150 / 150 | 170 / 150 |
| RBC-Test /L/D-Wert | 1.2 moderately irr. | 1.2 moderately irr. | 7.5 moderately/ slightly irr. | 1.0 moderately irr./irrit. |
| Viskosität / mPas | ~2000 | ~3000 | ~2000 | ~2000 |
| Kämmbarkeit | 4 | 4 | 4 | 4 |
| Haarvolumen | 3-4 | 3-4 | 3-4 | 3-4 |

Bewertung der Kämmbarkeit

1 - 3: schlecht durchzukämmen; das Haar setzt dem Kämmvorgang erheblichen Widerstand entgegen

4 - 7: das Haar läßt sich relativ gut durchkämmen, Kämmwiderstand verringert, je nach Haartyp ausreichend für ein Conditioning-Shampoo

8 - 10: vorbehalten für die nachfolgende Nachbehandlung durch sogenannte Hair-Rinse-Mittel

Bewertung des Haarvolumens und des Sitzes der Frisur

1 - 3: das Haar ist trocken oder strohig, schlechter Sitz der Frisur

4 - 7: das Haar ist weich und füllig, bei gleichzeitig gutem Sitz der Frisur

8 - 10: das Haar ist weich und glatt, jedoch zu locker, dadurch kein guter Sitz der Frisur.

Die Bewertung erfolgte nach einem abgestuften Punktesystem, wobei die oben angegebene Beurteilung den arithmetischen Mittelwert wiedergibt.

Tabelle 5

**Babyshampoo, mildes Haar- und Körpershampoo**

| Rezeptur | 1 | 2 | 3 |
|---|---|---|---|
| Beispiel 1 | 10 | - | - |
| Beispiel 5 | - | 10 | - |
| REWOTERIC® SBZ[1] | - | - | 10 |
| REWOTERIC® AM 2 C NM[9] | 15 | 15 | 15 |
| REWOPOL® NL 3-28[7] | 20 | 20 | 20 |
| REWODERM® LI S 80[5] | 3 | 3 | 3 |
| demin. Wasser | 52 | 52 | 52 |
| pH-Wert mit Citronensäure eingestellt | 6.5 | 6.5 | 6.5 |
| Anschaumvermögen | gut | gut | gut |
| Schaumstruktur | cremig | cremig | cremig |
| Hautgefühl nasse Haut | glatt | gut, etwas stumpf | stumpf, etw. klebrig |
| Hautgefühl trockene Haut<br>- sofort nach Abtrocknen<br>- nach kurzer Zeit | <br>gut<br>glatt, weich | <br>etw. stumpf, gut<br>glatt-sehr weich | <br>etw. stumpf, klebrig<br>glatt-sehr weich |
| Kämmbarkeit | 4 | 5 | 4 |
| Volumen des Haares<br>Sitz der Frisur | 3-4 | 4 | 4 |
| Viskosität / mPas | ~2000 | ~3000 | ~2000 |

Tabelle 6

| Hautreinigungsmittel und Make-up-Entferner für die empfindliche Haut | | | |
|---|---|---|---|
| **Rezeptur** | **1** | **2** | **3** |
| **Beispiel 1** | 10 | - | - |
| **Beispiel 5** | - | 10 | - |
| **REWOPOL® SB FA 30[6])** | - | - | 10 |
| **REWOTERIC® AM B 14[11])** | 13 | 13 | 13 |
| **demin. Wasser** | 77 | 77 | 77 |
| **pH-Wert, mit Citronensäure eingestellt** | 6,5 | 6,5 | 6,5 |
| **Zein-Werte / mgN / 100 ml (Hautverträglichkeit)** | 110 | 90 | 220 |

Der Einsatz der erfindungsgemäßen Sulfosuccinate in der vorliegenden Rezeptur ergibt ein sehr gutes hautverträgliches Reinigungsmittel, was sich im niedrigen Zein-Wert zeigt, wenn man als Vergleich die Rezeptur mit dem Standardsulfosuccinat REWOPOL® SB FA 30 nimmt.

Tabelle 7

**Hautfreundliches Geschirrspülmittel zur manuellen Anwendung, Reduzierung des Irritationspotentials**

| Rezeptur | 1 | 2 | 3 | 4 |
|---|---|---|---|---|
| Beispiel 1 | - | - | 7.5 | - |
| Beispiel 5 | - | 7.5 | - | - |
| REWOPOL® NL3-28 7) | 65 | 65 | 65 | 65 |
| REWOTERIC® AM CAS 3) | 3 | - | - | - |
| REWOPOL® LA 6 14) | 1.5 | - | - | 3 |
| demin. Wasser | 30.5 | 27.5 | 27.5 | 32 |
| Viskosität / mPas mit NaCl eingestellt auf ca. | 500 | 500 | 500 | 500 |
| Schaumvermögen / mm | 195/185 | 200/175 | 205/180 | 190/180 |
| + 0.5 ml Olivenöl | 190/180 | 195/170 | 200/175 | 190/180 |
| + 1.0 ml Olivenöl | 190/185 | 190/160 | 185/165 | 190/180 |
| Zein-Werte/mg N/100 ml | 250 | 240 | 250 | 270 |
| RBC-Test | 0.5 | 0.7 | 0.6 | 0.5 |

## Patentansprüche

**1.** Poly(oxyalkylen)alkanolaminfettsäureester der allgemeinen Formel (1)

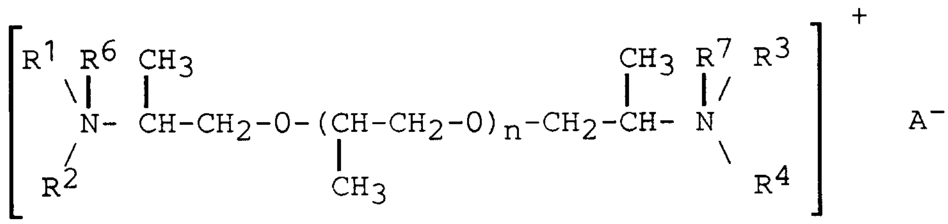

worin
$R^1$, $R^2$, $R^3$, $R^4$ gleich oder verschieden die Reste $R^5$-(O-$CH_2$-$CH_2$)$_m$- bedeuten mit
$R^5$ = gegebenenfalls substituierter, gegebenenfalls Mehrfachverbindungen enthaltender Acylrest mit 6 -22 mit
$R^5$ = Sulfosuccinatrest der Formeln

-C(O)-CH($SO_3^-X^+$)-CH2-$COO^-X^+$

oder

-C(O)-$CH_2$-CH-($SO_3^-X^+$)$COO^-X^+$

oder mit $R^5$ = H
und worin $R^5$ mindestens einmal ein Acylrest und mindestens einmal ein Sulfosuccinatrest ist, $R^6$, $R^7$ gleich oder verschieden H, -$CH_3$, -$C_2H_5$
sein können

- n = 2 - 8,
- m = 1 - 5 beträgt
- $A^-$ = mindestens ein organisches oder anorganisches Anion ist, wenn R6 und/oder R7 ungleich H ist,
- $X^+$ = H, Alkalimetall-, Ammonium-Ion ist.

**2.** Verfahren zur Herstellung der erfindungsgemäßen Poly(oxyalkylen)alkanolaminfettsäureester, welches dadurch gekennzeichnet ist, daß Aminverbindungen der allgemeinen Formel (2)

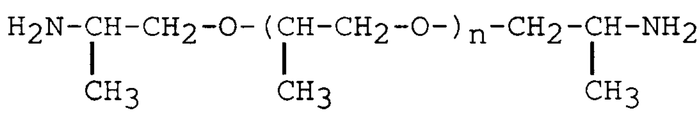
(2)

worin n = 2 - 8 ist, mit 4 - 20 mol Ethylenoxid bei 140 - 160 °C, gegebenenfalls unter Mitverwendung eines Katalysators, bei 1 - 4 bar in einem Druckreaktor zu Verbindungen der allgemeinen Formel (3)

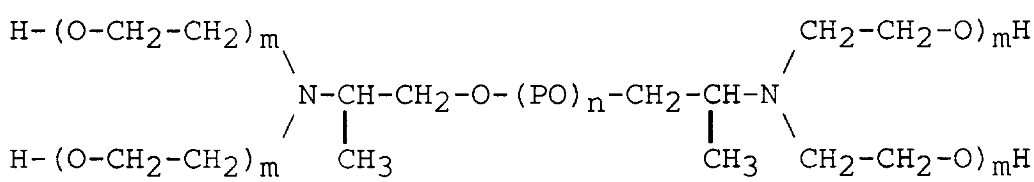

worin PO der Rest -CH($CH_3$)-$CH_2$-O-ist und n und m die oben angegebene Bedeutung haben, umgesetzt werden und mit ≧1 Mol Fettsäure(alkylester) pro Mol der Verbindung Formel (3) bei 160 - 240 °C verestert und das sich bildende Kondensationswasser bzw. der Alkohol kontinuierlich abdestilliert, gegebenenfalls zur Vervollständigung der Reaktion ein Unterdruck angelegt wird und dieser Ester

in einer Folgereaktion mit ≥1 Mol Maleinsäureanhydrid bei 70 - 80 °C zu dem entsprechenden Maleinsäurehalbester umgesetzt wird, welches in letzter Stufe mit 1 Equivalent, bezogen auf Maleinsäurehalbester, wäßriger $X_2SO_3$-Lösung, worin X die Bedeutung Alkalimetall oder Ammonium hat, bei 60 - 80 °C zum Sulfobernsteinsäurehalbester reagiert.

**3.** wäßrige Zubereitungen, enthaltend

a) 1 - 10 Gew.-Teile mindestens einer der Verbindungen der allgemeinen Formel (1)

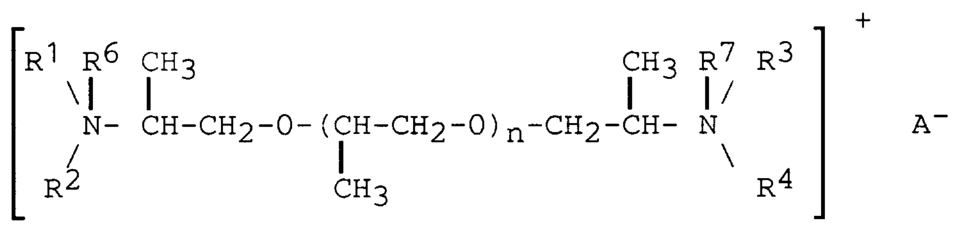

worin

$R^1$, $R^2$, $R^3$, $R^4$ gleich oder verschieden die Reste $R^5$-$(O-CH_2-CH_2)m$- bedeuten mit

$R^5$ = gegebenenfalls substituierter, gegebenenfalls Mehrfachverbindungen enthaltender Acylrest mit 6 - 22 vorzugsweise 8 - 18 C-Atomen, mit

$R^5$ = Sulfosuccinatrest der Formeln

$-C(O)-CH(SO_3-X+)-CH_2-COO-X+$

oder

$-C(O)-CH_2-CH(SO_3^-X^+)-COO^-X^+$

oder mit $R^5$ = H

und worin $R^5$ mindestens einmal ein Acylrest und mindestens einmal ein Sulfosuccinatrest ist, $R^6$, $R^7$ gleich oder verschieden H, $-CH_3$, $-C_2H_5$

sein können

n = 2 - 8,

m = 1 - 5 beträgt,

$A^-$ = mindestens ein organisches oder anorganisches Anion ist, wenn $R^6$ und/oder $R^7$ ungleich H ist,

$X^+$ = H, Alkalimetall-, Ammonium-Ion ist,

b) 1 - 20 Gew.-Teile, vorzugsweise 2 - 16 Gew.-Teile, mindestens eines Tensids aus der Gruppe der nichtionischen, amphoteren, zwitterionischen, ionischen Tenside und gegebenenfalls

c) 0,1 - 10 Gew.-Teile Verdickungsmittel, Duftstoffe, Pflanzenextrakte und sonstige Zusatz- und Hilfsstoffe und

d) ad 100 Wasser.

**4.** Babyshampoos, enthaltend 1 - 10 Gew.-Teile mindestens einer der Verbindungen der allgemeinen Formel (1).

**5.** Wäßrige Haarreinigungsmittel, enthaltend 1 - 10 Gew.-Teile mindestens einer der Verbindungen der allgemeinen Formel (1).

Europäisches Patentamt

# EUROPÄISCHER RECHERCHENBERICHT

Nummer der Anmeldung
EP 93 11 8701

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | DD-A-253 615 (AKADEMIE DER WISSENSCHAFTEN DER DDR)<br>* Seite 2, Zeile 8 - Zeile 10; Anspruch 1 * | 1,3-5 | C07C309/17<br>A61K7/075 |
| A | DE-A-22 58 564 (COLGATE-PALMOLIVE CO.)<br>* Ansprüche 4,9,10,14 * | 1,3 | |
| A | US-A-4 310 683 (K.D. LONGLEY ET AL)<br>* Anspruch 1 * | 1 | |

| RECHERCHIERTE SACHGEBIETE (Int.Cl.5) |
|---|
| C07C<br>A61K |

Der vorliegende Recherchenbericht wurde für alle Patentansprüche erstellt

| Recherchenort | Abschlußdatum der Recherche | Prüfer |
|---|---|---|
| BERLIN | 14. April 1994 | Kapteyn, H |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)